(19) **Europäisches Patentamt**
**European Patent Office**
**Office européen des brevets**

(11) **EP 2 604 170 B1**

(12) **EUROPEAN PATENT SPECIFICATION**

(45) Date of publication and mention
of the grant of the patent:
**03.08.2016 Bulletin 2016/31**

(51) Int Cl.:
*A61B 1/00* (2006.01)      *A61B 1/04* (2006.01)
*H04N 7/18* (2006.01)

(21) Application number: **11849237.0**

(22) Date of filing: **06.10.2011**

(86) International application number:
**PCT/JP2011/073110**

(87) International publication number:
**WO 2012/081297 (21.06.2012 Gazette 2012/25)**

(54) **ENDOSCOPIC APPARATUS**

ENDOSKOPVORRICHTUNG

APPAREIL ENDOSCOPIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2010   JP 2010282170**

(43) Date of publication of application:
**19.06.2013   Bulletin 2013/25**

(73) Proprietor: **Olympus Corporation
Shibuya-ku
Tokyo (JP)**

(72) Inventors:
 • **IGARASHI Makoto
Tokyo 151-0072 (JP)**

 • **YAMAZAKI Kenji
Tokyo 151-0072 (JP)**

(74) Representative: **Gunzelmann, Rainer
Wuesthoff & Wuesthoff
Patentanwälte PartG mbB
Schweigerstraße 2
81541 München (DE)**

(56) References cited:
**EP-A1- 1 787 577        JP-A- 5 244 608
JP-A- H05 244 608      JP-A- 2006 061 621
JP-A- 2006 095 330     JP-A- 2008 036 035
US-A1- 2001 007 921**

Printed by Jouve, 75001 PARIS (FR)

## Description

Technical Field

The present invention relates to an endoscope apparatus and a method of displaying an object image using an endoscope and, more particularly, to an endoscope apparatus and a method of displaying an object image using an endoscope that can clearly display a blood vessel in a deep portion of a mucous membrane.

Background Art

Document EP 1 787 577 A1 relates to an endoscope apparatus for capturing an image of living tissue and performing signal processing. An endoscope apparatus according to a second embodiment comprises a light-source apparatus for supplying an illumination light that covers the wavelength region of visible light, i.e. red, green and blue. The illumination passes through a narrowband filter. The narrowband filter comprises a narrowband transmittance filter characteristic portion Ra, Ga, Ba for each of the wavelength regions of red, green and blue. More specifically, the narrowband transmittance filter characteristic portions Ra, Ga, Ba have band pass characteristics, in which the respective center wavelengths are 600nm, 540nm and 420nm, and the full widths at half maximum are between 20 and 40nm. Illumination light is outputted to the outside, illuminating the surface of the living tissue of a diseased part or the like of a body cavity. An objective lens forms an optical image via the light reflected from the living tissue, and a charge-coupled device CCD is positioned at the image-forming location of the objective lens as a solid-state image-capturing device, and the image is subjected to photoelectric transfer by the CCD. The CCD inputs an image-capturing signal, which has undergone photoelectric transfer, to a CDS circuit. After a signal component has been extracted from the image-capturing signal, and converted to a baseband signal by the CDS circuit, the baseband signal is inputted to a Y/C separation/synchronization circuit, which performs Y/C separation and synchronization.

Conventionally, in a medical field, various low-invasive tests and surgeries employing endoscopes have been performed. A surgeon can insert an endoscope into a body cavity, observe an image of an object picked up by an image pickup device provided at a distal end portion of an insertion section of the endoscope, and, when necessary, apply treatment to a lesioned part using a treatment instrument inserted through a treatment instrument channel. Surgeries employing endoscopes have an advantage that a physical burden on a patient is small because laparotomy and the like are not performed.

An endoscope apparatus includes an endoscope, an image processing apparatus connected to the endoscope, and an observation monitor. An image of a lesioned part is picked up by an image pickup device provided at a distal end portion of an insertion portion of the endoscope. The image is displayed on the monitor. A surgeon can perform diagnosis or necessary treatment while looking at the image displayed on the monitor.

Some endoscope apparatus can perform not only a normal observation using white light but also a special light observation using special light such as infrared light in order to observe blood vessels inside a body.

In the case of an infrared endoscope apparatus, for example, indocyanine green (ICG) having a characteristic of an absorption peak in near infrared light near a wavelength of 805 nm is injected into blood of a patient as a drug. Infrared lights near the wavelength of 805 nm and near a wavelength of 930 nm from a light source device irradiate an object in a time division manner. A signal of an object image picked up by a CCD is inputted to a processor of the infrared endoscope apparatus. Concerning such an infrared endoscope apparatus, as disclosed in Japanese Patent Application Laid-Open Publication No. 2000-41942, an apparatus is proposed in which a processor allocates an image near the wavelength of 805 nm to a green signal (G), allocates an image near the wavelength of 930 nm to a blue signal (B), and outputs the images to a monitor (see, for example, Patent Literature 1). Since the image of the infrared light near the wavelength of 805 nm well-absorbed by the ICG is allocated to green, the surgeon can observe an infrared image during ICG administration at high contrast.

For example, in submucosal dissection (hereinafter referred to as ESD (endoscopic submucosal dissection)) for dissecting a submucosa in which a lesioned part is present, not to cut a relatively thick blood vessel in a mucous membrane with an electric knife or the like, the surgeon checks a position of the blood vessel and performs treatment such as dissection. A blood vessel that is likely to cause serious bleeding runs from the submucosa to a muscularis propria. When serious bleeding occurs in a manipulation such as the ESD, the surgeon has to perform bleeding stop work every time the bleeding occurs. Therefore, surgery time is increased.

However, in order to confirm the position of the blood vessel using the above-mentioned infrared endoscope apparatus, a cumbersome operation of an intravenous injection of a drug such as ICG is required, as mentioned above.

Further, in the case of the infrared endoscope apparatus, there is also a problem that the blood vessel in the image becomes blurred because the wavelength of the illumination light is a wavelength of the near infrared light.

Therefore, the present invention has been made in view of the above problem and an object of the present invention is to provide an endoscope apparatus and a method of displaying an object image using an endoscope, which are capable of clearly displaying a blood vessel in a deep portion of a mucous membrane without performing a cumbersome operation of drug administration.

Disclosure of Invention

Means for Solving the Problem

One or more of the above stated problems is solved by an endoscope apparatus according to claim 1.

A method of displaying an object image using an endoscope according to an aspect of the present invention, comprises: illuminating, by an irradiating section, an object to be examined with illumination light; receiving, by an image pickup section, reflected light of the illumination light irradiated to the object to be examined by the illuminating section and picking up an image of the object to be examined; generating, by an image signal processing section, based on image pickup signals obtained by the image pickup section, a first image signal with a high-frequency component suppressed, from an average image of image pickup signals of at least two or more narrow band wavelengths; generating, by the image signal processing section, a second image signal with a high-frequency component suppressed, from a difference image of image pickup signals of predetermined two narrow band wavelengths; and allocating, by a display section, the first image signal and the second image signal to one or more predetermined color channels and performs display.

Brief Description of the Drawings

Fig. 1 is a configuration diagram showing a configuration of an endoscope apparatus according to a first embodiment of the present invention;

Fig. 2 is a diagram showing a configuration of a rotating filter 14 according to the first embodiment of the present invention;

Fig. 3 is a diagram for explaining a flow of overall processing in a narrow band observation according to the first embodiment of the present invention;

Fig. 4 is a block diagram showing contents of processing by an image processing section 101 according to the first embodiment of the present invention;

Fig. 5 is a graph showing an example of respective filter characteristics of a band-pass filter 72 and a low-pass filter 73 according to the first embodiment of the present invention;

Fig. 6 is a diagram for explaining a displayed narrow band image according to the first embodiment of the present invention;

Fig. 7 is a configuration diagram showing a configuration of an endoscope apparatus 1A according to a modification 2 of the first embodiment of the present invention;

Fig. 8 is a block diagram showing a configuration of an image processing section 101A according to the modification 2 of the first embodiment of the present invention;

Fig. 9 is a diagram showing a configuration of a rotating filter according to a second embodiment of the present invention;

Fig. 10 is a block diagram showing contents of processing by an image processing section 101B according to the second embodiment of the present invention; and

Fig. 11 is a graph showing an example of respective filter characteristics of the band-pass filter 72, the low-pass filter 73, and a structure enhancing section 75 according to the second embodiment of the present invention.

Best Mode for Carrying Out the Invention

Embodiments of the present invention are explained below with reference to the accompanying drawings.

First Embodiment

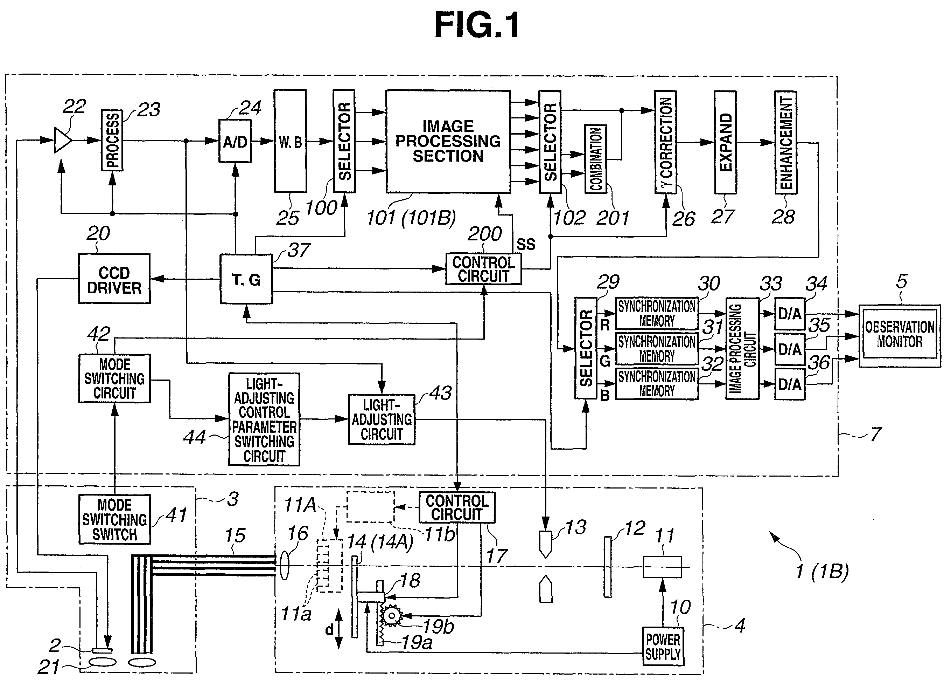

First, a configuration of an endoscope apparatus according to a first embodiment is explained. Fig. 1 is a configuration diagram showing the configuration of the endoscope apparatus according to the embodiment.

As shown in Fig. 1, an endoscope apparatus 1 according to the embodiment includes an electronic endoscope 3 including a CCD 2, which is an image pickup device, as living body image information acquiring means that is inserted into a body cavity and picks up an image of an intra-body cavity tissue, a light source device 4 that supplies illumination light to the electronic endoscope 3, and a video processor 7 that subjects an image pickup signal from the CCD 2 of the electronic endoscope 3 to signal processing and displays an endoscopic image on an observation monitor 5. The endoscope apparatus 1 has two modes: a normal light observation mode and a narrow band light observation mode. In the following explanation, the normal light observation mode of the endoscope apparatus 1 is the same as the normal light observation mode in the past. Therefore, explanation of a configuration of the normal light observation mode is simplified and the narrow band light observation mode is mainly explained.

The CCD 2 configures an image pickup section or image pickup means that receives reflected light of illumination light irradiating an object to be examined and picks up an image of the object to be examined.

The light source device 4 includes a xenon lamp 11 functioning as illuminating means that emits illumination light (white light), a heat ray cut filter 12 that cuts a heat ray of the white light, a diaphragm device 13 that controls a light amount of the white light passed through the heat ray cut filter 12, a rotating filter 14 functioning as band limiting means that changes the illumination light to frame-sequential light, a condensing lens 16 that condenses the frame-sequential light passed through the rotating filter 14 on an incident surface of a light guide 15 disposed in the electronic endoscope 3, and a control circuit 17 that controls rotation of the rotating filter 14. The xenon lamp 11, the rotating filter 14, and the light guide 15 configure an irradiating section or irradiating means that illuminates the object to be examined with the illumination light.

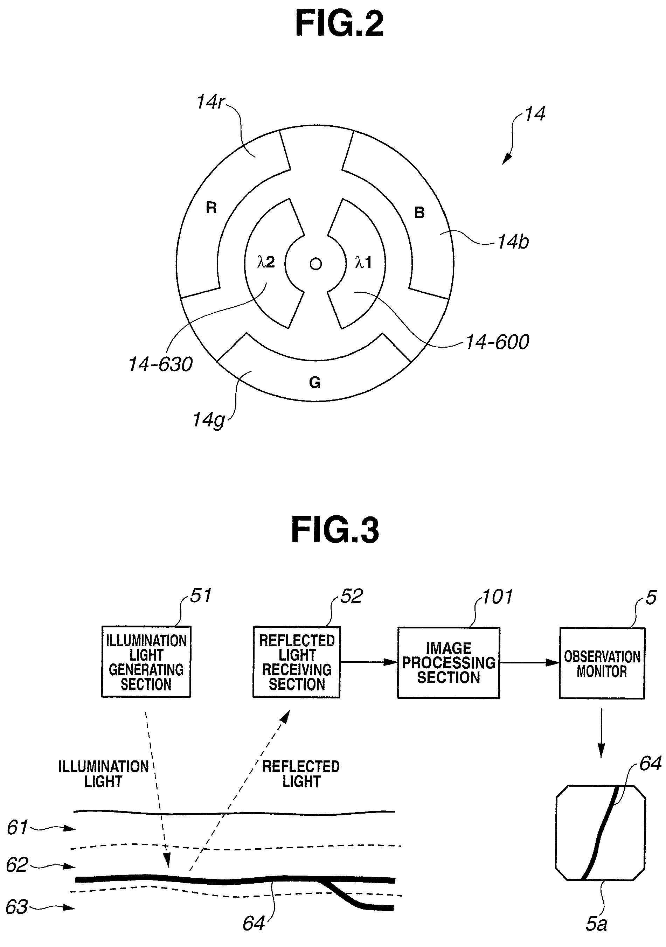

Fig. 2 is a diagram showing a configuration of the rotating filter 14. As shown in Fig. 2, the rotating filter 14 functioning as a wavelength band limiting section or wavelength band limiting means is formed in a disc shape and in structure having a rotating shaft in a center. The rotating filter 14 includes two filter groups. On an outer circumferential side of the rotating filter 14, an R (red) filter section 14r, a G (green) filter section 14g, and a B (blue) filter section 14b forming a filter set for outputting frame-sequential light having a spectral characteristic for normal observation are arranged along a circumferential direction as a first filter group.

On an inner circumferential side of the rotating filter 14, two filters 14-600 and 14-630 that transmit lights having two predetermined narrow band wavelengths are arranged along the circumferential direction as a second filter group.

The filter 14-600 is configured to transmit light near a wavelength of 600 nm as narrow band light. The filter 14-630 is configured to transmit light near a wavelength of 630 nm as the narrow band light.

In the embodiment, as the narrow band light, lights in a red band of a visible region and near the wavelength of 600 nm and near the wavelength of 630 nm where a hemoglobin light absorption characteristic is suddenly attenuated are used. In the case of near the wavelength of 600 nm, "near" means that the light is narrow band light having a center wavelength of 600 nm and having a distribution in a range of width of, for example, 20 nm around the wavelength of 600 nm (i.e., a wavelength of 590 nm to a wavelength of 610 nm before and after the wavelength of 600 nm). The same holds true for other wavelengths: the wavelength of 630 nm and a wavelength of 540 nm explained below.

The rotating filter 14 is arranged on an optical path extending from the xenon lamp 11, which is an emitting section of the illumination light, to an image pickups surface of the CCD 2. The rotating filter 14 limits at least two wavelength bands among plural wavelength bands of the illumination light to be narrowed.

The control circuit 17 controls a motor 18 for rotating the rotating filter 14 and controls the rotation of the rotating filter 14.

A rack 19a is connected to the motor 18. A not-shown motor is connected to a pinion 19b. The rack 19a is attached to be screwed with the pinion 19b. The control circuit 17 can move the rotating filter 14 in a direction indicated by an arrow d by controlling rotation of the motor connected to the pinion 19b. Therefore, the control circuit 17 selects the first filter group or the second filter group according to mode switching operation by a user explained below.

Electric power is supplied from a power supply section 10 to the xenon lamp 11, the diaphragm device 13, the rotating filter motor 18, and the motor (not shown) connected to the pinion 19b.

The video processor 7 includes a CCD driving circuit 20 functioning as a CCD driver, an amplifier 22, a process circuit 23, an A/D converter 24, a white balance circuit (hereinafter referred to as W. B) 25, a selector 100, an image processing section 101, a selector 102, a $\gamma$ correction circuit 26, an expansion circuit 27, an enhancement circuit 28, a selector 29, synchronization memories 30, 31, and 32, an image processing circuit 33, D/A converters 34, 35, and 36, a timing generator (hereinafter referred to as T.G) 37, a control circuit 200, and a combining circuit 201 functioning as display image generating means.

The CCD driving circuit 20 drives the CCD 2 provided in the electronic endoscope 3 and outputs a frame-sequential image pickup signal that synchronizes with the rotation of the rotating filter 14. The amplifier 22 amplifies a frame-sequential image pickup signal obtained by picking up an image of an intra-body cavity tissue with the CCD 2 via an objective optical system 21 provided at a distal end of the electronic endoscope 3.

The process circuit 23 applies correlated double sampling, noise removal, and the like to the frame-sequential image pickup signal via the amplifier 22. The A/D converter 24 converts the frame-sequential image pickup signal having passed through the process circuit 23 into a frame-sequential image signal of a digital signal.

The W. B 25 performs gain adjustment and executes white balance processing on the frame-sequential image signal digitized by the A/D converter 24 such that, for example, brightness of an R signal of the image signal and brightness of a B signal of the image signal are equal with reference to a G signal of the image signal.

The selector 100 distributes the frame-sequential image signal from the W. B 25 to sections in the image processing section 101 and outputs the frame-sequential image signal.

The image processing section 101 is an image signal processing section or image signal processing means that converts RGB image signals for normal light observation or two image signals for narrow band light observation from the selector 100 into an image signal for display. The image processing section 101 outputs, according to a selection signal SS from the control circuit 200 based on a mode signal, image signals during the normal light observation mode and during the narrow band light observation mode to the selector 102.

The selector 102 sequentially outputs a frame-sequential image signal of the image signal for normal light observation and the image signal for narrow band light observation from the image processing section 101 to the γ correction circuit 26 and the combining circuit 201.

The γ correction circuit 26 applies γ correction processing to the frame-sequential image signal from the selector 102 or the combining circuit 201. The expansion circuit 27 applies expansion processing to the frame-sequential image signal subjected to the γ correction processing by the γ correction circuit 26. The enhancement circuit 28 applies edge enhancement processing to the frame-sequential image signal subjected to the expansion processing by the expansion circuit 27. The selector 29 and the synchronization memories 30, 31, and 32 are sections for synchronizing the frame-sequential image signal from the enhancement circuit 28.

The image processing circuit 33 reads out frame-sequential image signals stored in the synchronization memories 30, 31, and 32 and performs moving image color shift correction processing and the like. The D/A converters 34, 35 and 36 convert the image signal from the image processing circuit 33 into RGB analog video signals and output the RGB analog video signals to the observation monitor 5. The T. G 37 receives input of a synchronization signal, which synchronizes with the rotation of the rotating filter 14, from the control circuit 17 of the light source device 4 and outputs various timing signals to respective circuits in the video processor 7.

In the electronic endoscope 2, a mode switching switch 41 for switching of the normal light observation mode and the narrow band light observation mode is provided. An output of the mode switching switch 41 is outputted to the mode switching circuit 42 in the video processor 7. The mode switching circuit 42 of the video processor 7 outputs a control signal to a light-adjusting control parameter switching circuit 44 and the control circuit 200. The light-adjusting circuit 43 controls the diaphragm device 13 of the light source device 4 and performs proper brightness control based on light-adjusting control parameters from the light-adjusting control parameter switching circuit 44 and an image pickup signal processed by the process circuit 23.

The respective circuits in the video processor 7 execute predetermined processing corresponding to the designated mode. Processing corresponding to each of the normal light observation mode and the narrow band light observation mode is executed. An image for normal light observation or an image for narrow band light observation is displayed on the observation monitor 5.

An overall rough flow of the narrow band observation in the embodiment is briefly explained below.

Fig. 3 is a diagram for explaining the flow of the overall processing in the narrow band observation in the embodiment.

The surgeon inserts the insertion section of the endoscope into a body cavity and locates the distal end portion of the insertion section of the endoscope near a lesioned part under the normal observation mode. When the surgeon confirms the lesioned part to be treated, to observe a relatively thick blood vessel having a diameter of 1 to 2 mm, and running from a submucosa to a muscularis propria the surgeon operates the mode switching switch 41 and switches the endoscope apparatus 1 to the narrow band observation mode.

Under the narrow band observation mode, the control circuit 17 of the endoscope apparatus 1 controls the motor connected to the pinion 19b and moves the position of the rotating filter 14 to emit light transmitted through the second filter group from the light source device 4. Further, the control circuit 200 also controls the various circuits in the video processor 7 to perform image processing for observation by narrow band wavelength.

As shown in Fig. 3, in the narrow band mode, illumination light having narrow band wavelength from an illumination light generating section 51 is emitted from the distal end portion of the insertion section of the endoscope 3, transmitted through a stratum mucosum 61, and irradiates a blood vessel 64 running in a submucosa 62 and a muscularis propria 63. The illumination light generating section 51 includes the light source device 4, the rotating filter 14, the light guide 15, and the like and emits illumination light from the distal end of the insertion section of the endoscope. According to the rotation of the rotating filter 14, narrow band light near the wavelength of 600 nm and narrow band light near the wavelength of 630 nm are alternately emitted from the light source device 4 to irradiate an object.

Reflected lights of the narrow band light near the wavelength of 600 nm and the narrow band light near the wavelength of 630 nm are received by a reflected light receiving section 52 functioning as the CCD 2. The CCD 2 outputs image pickup signals of the respective reflected lights. The image pickup signals are supplied to the selector 100 via the amplifier 22 and the like. The selector 100 keeps a first image P1 near the wavelength of 600 nm and a second image P2 near the wavelength of 630 nm and supplies the images to the image processing section 101 according to a predetermined timing from the T. G 37.

The image processing section 101 shown in Fig. 1 performs image processing explained later and supplies respective image signals obtained by the image processing to RGB channels of the observation monitor 5 via the selector 102 and the like. As a result, the relatively thick blood vessel 64 of 1 to 2 mm in a deep portion of a mucous membrane is displayed at high contrast on a screen 5a of the observation monitor 5. The surgeon can apply the ESD to the lesioned part while paying attention to the blood vessel 64 of 1 to 2 mm running in the submucosa 62 and the muscularis propria 63.

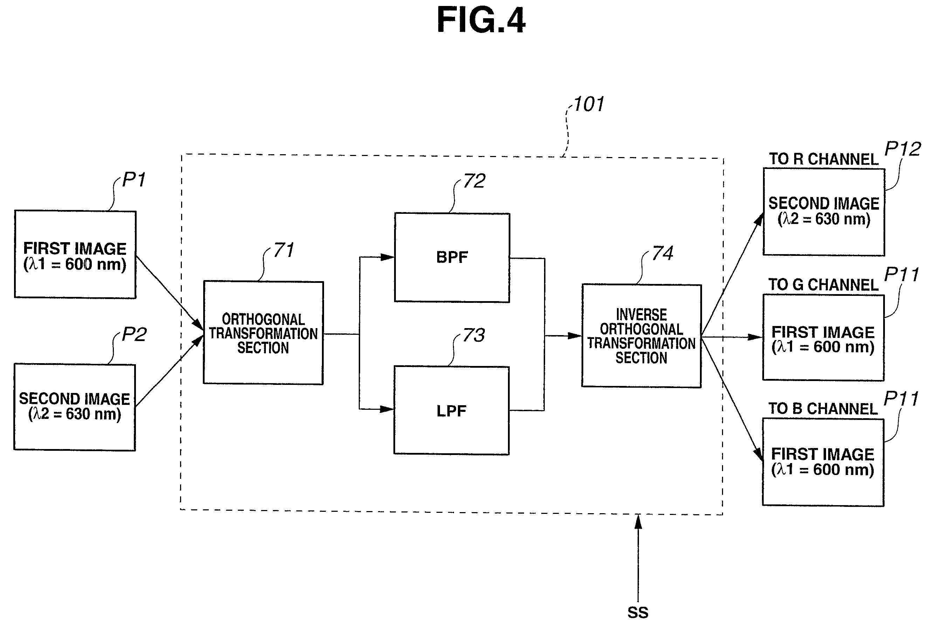

Fig. 4 is a block diagram showing contents of processing by the image processing section 101. The first image P1 near the wavelength of 600 nm and the second image P2 near the wavelength of 630 nm are inputted to the image processing section 101. An orthogonal transformation section 71 applies predetermined orthogonal transformation processing to the two images P1 and P2. In the following explanation, a processing circuit during the normal light observation mode is omitted and a processing circuit during the narrow band light observation mode is explained.

The orthogonal transformation section 71 executes orthogonal transformation indicated by Equation (1) below on the first and second images P1 and P2. The orthogonal transformation section 71 performs the orthogonal transformation of Equation (1) for each of corresponding pixels of the first and second images P1 and P2 to generate an average image PA and a difference image PS.

$$AX = Y \qquad \text{equation (1)}$$

where, A is a coefficient matrix indicated by Equation (2) below.

$$A = \begin{pmatrix} a1 & a2 \\ a3 & a4 \end{pmatrix} = \begin{pmatrix} 0.6 & 0.4 \\ 0.4 & -0.6 \end{pmatrix} \qquad \text{equation (2)}$$

X is a matrix of respective pixel values x1 and x2 in corresponding same positions in the first image P1 and the second image P2.

$$X = \begin{pmatrix} x1 \\ x2 \end{pmatrix} \qquad \text{equation (3)}$$

Y is a matrix of respective pixel values y1 and y2 in corresponding same positions in the average image PA and the difference image PS.

$$Y = \begin{pmatrix} y1 \\ y2 \end{pmatrix} \qquad \text{equation (4)}$$

In short, the coefficient matrix A is a matrix for generating the average image PA and the difference image PS from the first image P1 and the second image P2. As an example, coefficients a1, a2, a3, and a4 are 0.6, 0.4, 0.4, and -0.6. Each pixel value y1 of the average image PA is not a simple average of pixel values of the first image P1 and the second image P2 but is an average of weighted pixel values. Similarly, each pixel value y2 of the difference image PS is not a simple difference of the pixel values of the first image P1 and the second image P2 but is a difference value of the weighted pixel values.

The orthogonal transformation section 71 can generate various average images and various difference images by adjusting the respective coefficients of the coefficient matrix A.

The orthogonal transformation section 71 executes the calculation of Equation (1) above, generates the average image PA and the difference image PS from the first image P1 and the second image P2, and outputs the average image PA and the difference image PS respectively to a band-pass filter (BPF) 72 and a low-pass filter (LPF) 73. The average image PA is subjected to spatial filtering by the band-pass filter 72. The difference image PS is subjected to spatial filtering by the low-pass filter 73.

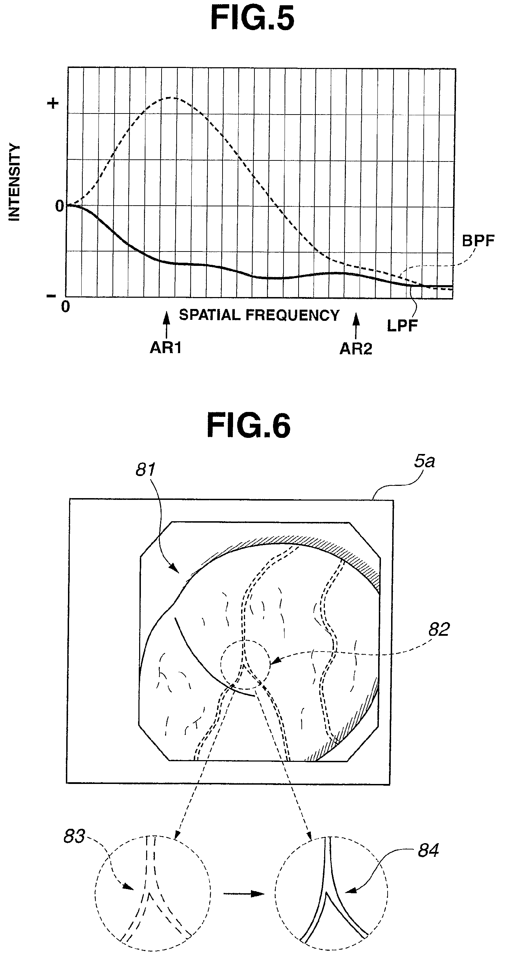

The band-pass filter 72 and the low-pass filter 73 are spatial filters that perform spatial filtering processing having characteristics shown in Fig. 5.

Fig. 5 is a graph showing an example of filter characteristics of the band-pass filter 72 and the low-pass filter 73. In Fig. 5, a solid line indicates the filter characteristic of the low-pass filter (LPF) and a dotted line indicates the filter characteristic of the band-pass filter (BPF). Fig. 5 is a graph in which an ordinate is an axis of intensity and an abscissa is an axis of a spatial frequency. In Fig. 5, a spatial frequency increases as the spatial frequency on the abscissa increases from 0 (i.e., to a right side of the abscissa in Fig. 5). A signal is increased as a value on the ordinate increases to be larger than 0 (i.e., to an upper side than 0 on the ordinate in Fig. 5) and is reduced as the value decreases to be smaller than 0 (i.e., to a lower side than 0 on the ordinate in Fig. 5).

As indicated by the dotted line, the band-pass filter 72 is a filter having a characteristic that a signal near a spatial frequency corresponding to a blood vessel of 1 to 2 mm indicated by an arrow AR1 is further enhanced and signals having frequencies lower than and higher than the spatial frequency are suppressed. A signal of a spatial frequency indicated by an arrow AR2 is further suppressed than the signal near the spatial frequency indicated by the arrow AR1.

As indicated by the solid line, the low-pass filter 73 has a characteristic that the signal of the higher spatial frequency indicated by the arrow AR2 than the signal near the spatial frequency corresponding to the blood vessel of 1 to 2 mm indicated by the arrow AR1 is further suppressed.

In short, the band-pass filter 72 and the low-pass filter 73 enhance images of the thick blood vessel of 1 to 2 mm in the average image PA and the difference image PS near the wavelength of 600 nm and near the wavelength of 630 nm and suppress images of thin blood vessels such as capillary vessels.

The filter characteristics of the band-pass filter 72 and the low-pass filter 73 are not limited to the characteristics shown in Fig. 5. As explained above, the filter characteristics only have to be characteristics that the images of the thick blood vessel of 1 to 2 mm are enhanced and the images of the thin blood vessels such as capillary blood vessels are suppressed.

As explained above, the image processing section 101 includes two spatial filters. The band-pass filter 72 suppresses a high-frequency component and generates a first image signal from an average image. The low-pass filter 73 suppresses a high-frequency component and generates a second image signal from a difference image. Specifically, based on an image pickup signal obtained by the CCD 2, the image processing section 101 generates a first image signal with a high-frequency component suppressed from an average image of image pickup signals having at least two or more narrow band wavelengths and generates a second image signal with a high-frequency component suppressed from a difference image of image pickup signals having predetermined two narrow band wavelengths.

The images respectively processed by the band-pass filter 72 and the low-pass filter 73 are supplied to an inverse orthogonal transformation section 74. The inverse orthogonal transformation section 74 executes inverse orthogonal transformation processing for the images.

Inverse orthogonal transformation in the inverse orthogonal transformation section 74 is executed using Equation (5) below. The inverse orthogonal transformation section 74 generates a first image P11 near the wavelength of 600 nm and a second image P12 near the wavelength of 630 mm. The inverse orthogonal transformation section 74 performs inverse orthogonal transformation of Equation (5) for each corresponding pixel of the images respectively processed by the band-pass filter 72 and the low-pass filter 73. The inverse orthogonal transformation section 74 generates the first image P11 near the wavelength of 600 nm and the second image P12 near the wavelength of 630 nm.

$$X=BY=A^{-1}Y \qquad \text{equation (5)}$$

where, B is an inverse matrix of A.

As explained above, the image processing section 101 generates an average image and a difference image through the orthogonal transformation processing for image signals having at least two or more narrow band wavelengths and generates a first image signal and a second image signal through the inverse orthogonal transformation processing for respective images signals of the average image and the difference image. The image processing section 101 includes the orthogonal transformation section 71 and the inverse orthogonal transformation section 74. The orthogonal transformation section 71 performs the orthogonal transformation processing through matrix calculation using a coefficient matrix. The inverse orthogonal transformation section 74 performs the inverse orthogonal transformation processing through matrix calculation using an inverse matrix of the coefficient matrix.

The inverse orthogonal transformation section 74 allocates the generated image P11 near the wavelength of 600 nm to G and B channels, allocates the image P12 near the wavelength of 630 nm to an R channel, and outputs the images P11 and P12.

The images processed by the image processing section 101 is stored in the synchronization memories 30, 31, and 32 of the corresponding channels of RGB after being subjected to γ correction and the like and then subjected to D/A conversion and outputted to the observation monitor 5.

The selector 102 and the observation monitor 5 configure a display section that allocates the first image signal and the second image signal to one or more predetermined color channels and performs display.

Fig. 6 is a diagram for explaining a displayed narrow band image. As shown in Fig. 6, an object image is displayed in the screen 5a of the observation monitor 5. A relatively thick blood vessel having a diameter of, for example, 1 to 2 mm running from a submucosa to a muscularis propria is displayed at high contrast. For example, in Fig. 6, a blood vessel image 84 in a region 82 in a narrow band image 81 is an image clearer than a blurred blood vessel image 83 in the past.

The image near the wavelength of 630 nm is allocated to the R channel and the image near the wavelength of 600 nm is allocated to the G and B channels. Therefore, in the image displayed on the observation monitor 5, the blood vessel is displayed in red, which is a color close to pseudo color display. Consequently, for the surgeon, the image displayed on the observation monitor 5 looks like an image of a natural color.

Therefore, with the endoscope apparatus according to the embodiment explained above, the relatively thick blood vessel running from submucosa to the muscularis propria is displayed on the observation monitor 5 at high contrast. Consequently, the surgeon can accurately grasp a position of such a blood vessel and apply surgery such as the ESD.

In particular, in the case of the ESD, a peripheral portion of a lesioned part such as a cancer cell is dissected and peeled off by an electric knife to remove the lesioned part. However, in an endoscopic image in the past, visibility of the relatively thick blood vessel running from the submucosa to the muscularis propria is not high. In the past, there is also an endoscope apparatus that uses narrow band light having a wavelength of 415 nm or 540 nm. However, an image of a blood vessel at a depth of 1 to 2 mm cannot be picked up. When near-infrared light is used, an image of the blood vessel is blurred and contrast is low.

When the relatively thick blood vessel having the diameter of 1 to 2 mm running from the submucosa to the muscularis propria is cut, the cutting is likely to lead to massive bleeding. Therefore, surgery time of the ESD is long and stress on the surgeon is large.

On the other hand, with the endoscope apparatus according to the embodiment, a blood vessel in a deep portion of a mucous membrane is clearly displayed without complicated work of drug administration being performed. As a result, the visibility of the relatively thick blood vessel having the diameter of 1 to 2 mm running from the submucosa to the muscularis propria is improved. Therefore, it is possible to realize a reduction in surgery time and a reduction in stress on the surgeon.

Modifications of the embodiment are explained below.

Modification 1

The light source device 4 explained above generates illumination light in a desired wavelength band using the xenon lamp 11, the rotating filter 14, and the like. However, in an endoscope apparatus in a modification 1, as indicated by dotted lines, the light source device 4 includes a light emitting section 11A including a light emitting diode group 11a including plural light emitting diodes (LEDs) that emit lights having desired wavelengths, for example, wavelengths of RGB corresponding to the first filter group and wavelengths near 600 nm and near 630 nm corresponding to the second filter group. The light emitting section 11A and the light guide 15 configure an irradiating section that irradiates an object with illumination light.

For example, in Fig. 1, instead of the xenon lamp 11, the heat ray cut filter 12, the diaphragm device 13, the rotating filter 14, and the like, the light emitting section 11A indicated by the dotted line is provided in the light source device 4. Further, in the light source device 4, a driving circuit 11b for driving respective light emitting diodes of the light emitting section 11 A at predetermined timings according to respective modes is provided. The light emitting section 11A including plural LEDs 11a receives power from the power supply 10 and is controlled and driven by the driving circuit 11b under a control signal from the control circuit 17.

When the endoscope apparatus 1 is configured using the light source device according to the modification 1, effects same as the effects explained above can be obtained.

The light emitting section 11 A may include a laser diode (LD) that emits predetermined plural narrow band lights.

When an LED or the like is used as a light source and a CMOS sensor or the like is used as image pickup means, it is possible to display an image of the normal light observation mode and an image of the narrow band light observation mode on the screen 5a of the observation monitor 5 in parallel. In other words, the user can observe the image of the narrow band light observation mode without performing the switching operation by the mode switching switch 41.

Modification 2

In the embodiment and the modification 1 explained above, the predetermined narrow band light is generated by the rotating filter 14 or the light emitting device such as the predetermined light emitting diode. However, an endoscope apparatus 1A according to a modification 2 uses white light as illumination light from a light source, obtains a spectral image of predetermined narrow band light through spectral estimation processing, and executes the image processing explained above on the spectral image.

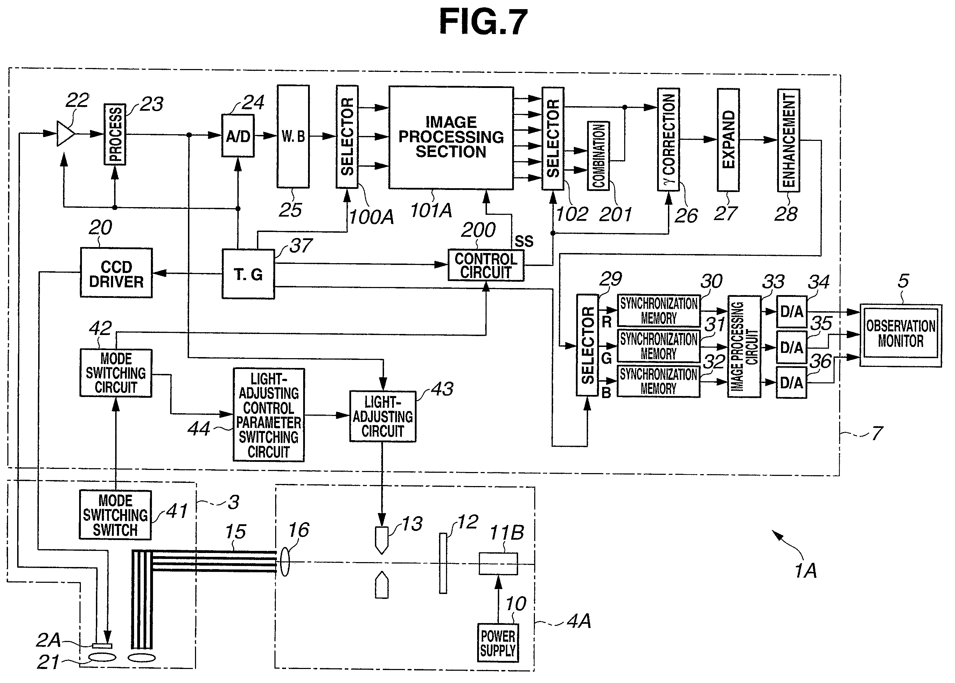

Fig. 7 is a configuration diagram showing a configuration of the endoscope apparatus 1A according to the modification 2. In Fig. 7, components same as the components shown in Fig. 1 are denoted by the same reference numerals and signs and explanation of the components is omitted.

As shown in Fig. 7, a light source device 4A includes a lamp 11B that emits white light, the heat ray cut filter 12, and the diaphragm device 13. Illumination light from the light source device 4A irradiates an object via the light guide 15.

An image pickup device 2A provided at the distal end of the insertion section of the endoscope 3 is a color image pickup device. The image pickup device 2A is, for example, a color CCD and includes RGB color filters on an image pickup surface. Reflected light from the object is received by respective pixel sections of the image pickup surface via the RGB color filters functioning as wavelength band limiting means. Image signals of three colors RGB are outputted from the image pickup device 2A.

A selector 100A outputs the three image signals of RGB to an image processing section 101A. The image processing section 101A includes a spectral estimation section. In narrow band light observation, the image processing section 101A outputs an image signal near the wavelength of 600 nm and an image signal near the wavelength of 630 nm.

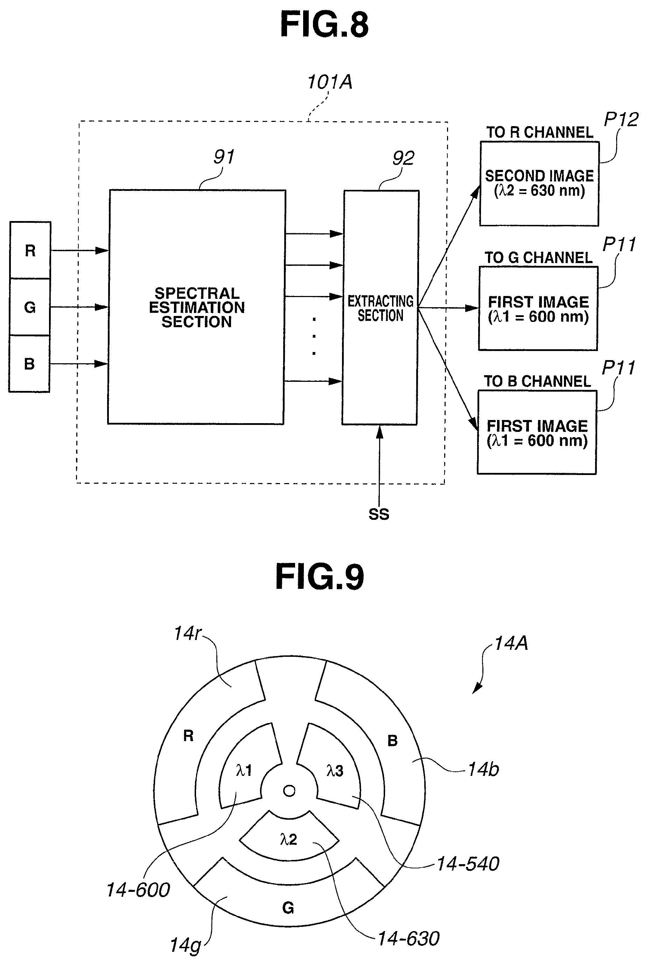

Fig. 8 is a block diagram showing a configuration of the image processing section 101A. The image processing section 101A includes a spectral estimation section 91 and an extracting section 92. The image processing section 101 A extracts spectral images of arbitrary wavelength components from spectral images obtained by spectral estimation processing and outputs the spectral images. Specifically, the image processing section 101 A extracts a first image near the wavelength of 600 nm and a second image near the wavelength of 630 nm from the three images of RGB, allocates the second image to the R channel, and allocates the first image to the G and B channels.

The spectral estimation section 101A calculates an n-dimensional spectral image through matrix calculation based on three inputs and outputs the calculated n-dimensional spectral image. The spectral estimation section 101A is configured to calculate, in the matrix calculation, n image signals including the image signal near the wavelength of 600 nm and the image signal near the wavelength of 630 nm and output the n image signals.

The n image signals from the spectral estimation section 101 A is supplied to the extracting section 92. The extracting section 92 selects, according to a selection signal SS from the control circuit 200 based on a mode signal, the image signal near the wavelength of 600 nm and the image signal near the wavelength of 630 nm from the n image signals and allocates the image signals to the RGB channels as explained above.

Processing thereafter applied to the first and second images outputted from the image processing section 101A is the same as the processing explained above.

Therefore, effects same as the effects of the endoscope apparatus 1 can be obtained by the endoscope apparatus 1A according to the modification 2 as well.

Second Embodiment

An endoscope apparatus according to a second embodiment is explained below.

In the endoscope apparatuses according to the first embodiment and the two modifications of the first embodiment, the two narrow band lights near the wavelength of 600 nm and near the wavelength of 630 nm are used as the narrow band light. The endoscope apparatus according to the second embodiment is different from the endoscope apparatus according to the first embodiment in that three narrow band lights are generated and allocated to RGB channels.

A configuration of an endoscope apparatus 1B according to the embodiment is substantially the same as the endoscope apparatus 1 according to the first embodiment. Therefore, differences from the endoscope apparatus 1 according to the first embodiment are explained. In the configuration shown in Fig. 1, the configuration of the endoscope apparatus according to the embodiment is different in a configuration of a rotating filter. Fig. 9 is a diagram showing the configuration of the rotating filter according to the embodiment. As shown in Fig. 9, a rotating filter 14A functioning as wavelength band limiting means is formed in a disc shape. Like the rotating filter 14 shown in Fig. 2, the rotating filter 14A has a structure having a rotating shaft in a center. The rotating filter 14A includes two filter groups. Like the rotating filter 14 shown in Fig. 2, on an outer circumferential side of the rotating filter 14A, an R filter section 14r, a G filter section 14g, and a B filter section 14b forming a filter set for outputting frame-sequential light having spectral characteristics for normal observation are arranged along a circumferential direction as a first filter group.

On an inner circumferential side of the rotating 14A, three filters 14-600, 14-630, and 14-540 for transmitting lights in three predetermined narrow band wavelengths are arranged along the circumferential direction as a second filter group.

The second filter group includes the filter 14-540 in addition to the filter 14-600 and the filter 14-630. The filter 14-540 is configured to transmit light having wavelength near 540 nm as narrow band light.

During a narrow band light observation mode, the rotating filter 14A emits light having wavelength near 540 nm, light having wavelength near 600 nm, and light having wavelength near 630 nm in order as narrow band lights.

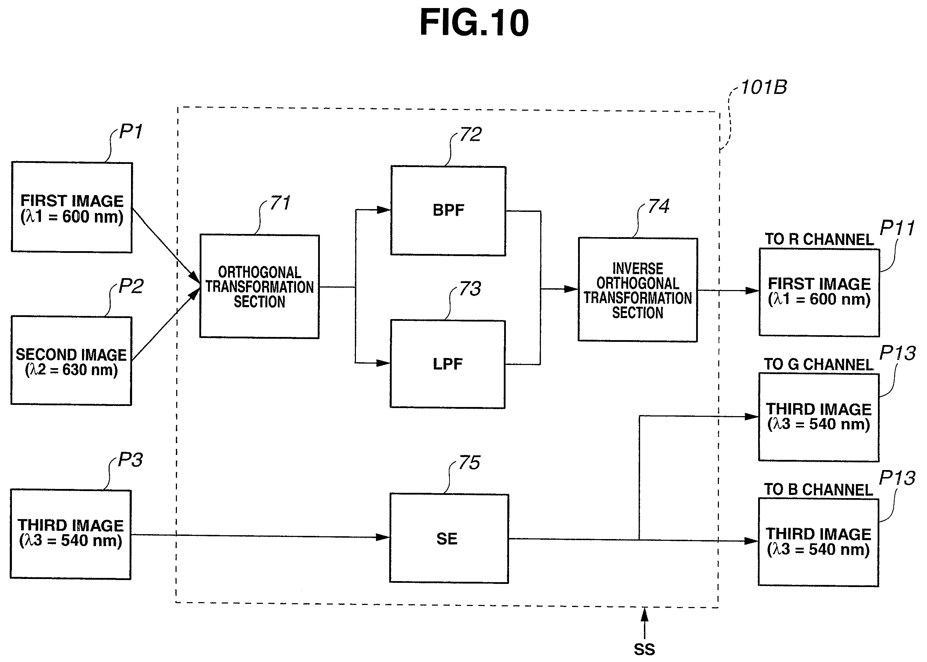

Fig. 10 is a block diagram showing contents of processing by the image processing section 101B according to the second embodiment. The image processing section 101B includes the orthogonal transformation section 71, the band-pass filter 72, the low-pass filter 73, the inverse orthogonal transformation section 74, and the structure enhancing section 75.

Kinds of processing by the orthogonal transformation section 71, the band-pass filter 72, the low-pass filter 73, and the inverse orthogonal transformation section 74 are the same as the kinds of processing in the first embodiment. However, the second embodiment is different from the first embodiment in that only the first image P11 near the wavelength of 600 nm of the two generated images of the inverse orthogonal transformation section 74 is allocated to a color channel.

The structure enhancing section 75 applies structure enhancement processing to an image signal near the wavelength of 540 nm. Sharpness of an image near the wavelength of 540 nm is increased by the structure enhancement processing. The structure enhancing section 75 generates the image signal near the wavelength of 540 nm subjected to the structure enhancement processing. The structure enhancing section 75 of the image processing section 101B executes the structure enhancement processing on a third image signal in a wavelength band shorter than the two narrow band wavelengths (near the wavelength of 600 nm and near the wavelength of 630 nm).

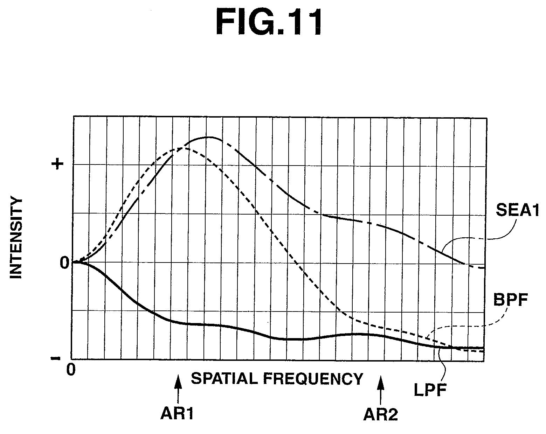

Fig. 11 is a graph showing an example of respective filter characteristics of the band-pass filter 72, the low-pass filter 73, and the structure enhancing section 75. In Fig. 11, a solid line indicates the filter characteristic of the low-pass filter (LPF), a dotted line indicates the filter characteristic of the band-pass filter (BPF), and an alternate long and short dash line indicates the filter characteristic of the structure enhancing section 75.

The respective filter characteristics of the band-pass filter 72 and the low-pass filter 73 are the same as the characteristics shown in Fig. 5. The filter characteristic of the structure enhancing section 75 is a characteristic that, as indicated by the alternate long and short dash line (SEA1), a signal near a spatial frequency corresponding to a blood vessel having a diameter of 1 to 2 mm indicated by an arrow A1 is further enhanced but a degree of enhancement for signals having frequencies lower than and higher than the spatial frequency is lower than a degree of enhancement of the signal near the spatial frequency corresponding to the blood vessel having the diameter of 1 to 2 mm. For example, a degree of enhancement of a signal having a spatial frequency indicated by an arrow AR2 is lower than the degree of enhancement of the signal near the spatial frequency indicated by the arrow AR1.

The first image P1 near the wavelength of 600 nm and the second image P2 near the wavelength of 630 nm are inputted to the image processing section 101B. The orthogonal transformation section 71 applies predetermined orthogonal transformation processing to the two images P1 and P2.

The average image PA and the difference image PS generated by the orthogonal transformation section 71 are respectively outputted to the band-pass filter (BPF) 72 and the low-pass filter (LPF) 73. The respective images processed by the band-pass filter 72 and the low-pass filters 73 are supplied to the inverse orthogonal transformation section 74. The inverse orthogonal transformation section 74 executes inverse orthogonal transformation processing on the respective images.

The inverse orthogonal transformation section 74 generates the first image P11 near the wavelength of 600 nm and the second image P12 near the wavelength of 630 nm. Processing contents of the orthogonal transformation section 71, the band-pass filter 72, the low-pass filter 73, and the inverse orthogonal transformation section 74 are the same as the processing contents in the first embodiment.

As explained above, the display section including the selector 102 and the observation monitor 5 allocates the image signal near the wavelength of 540 nm serving as a third image signal and one image signal of the first image signal and the second image signal to one or more predetermined color channels and displays the image signals. Specifically, in the selector 102 and the observation monitor 5, the first image P11 near the wavelength of 600 nm generated in the inverse orthogonal transformation section 74 is allocated to the R channel and the image near the wavelength of 540 subjected to structure enhancement in the structure enhancing section 75 is allocated to the G and B channels.

With the endoscope apparatus 1B according to this embodiment, a relatively thick blood vessel running from the submucosa to the muscularis propria is displayed on the observation monitor 5 at high contrast without complicated work of drug administration being performed. Consequently, the surgeon can accurately grasp a position of such a blood vessel and apply surgery such as the ESD.

Monochrome images picked up at respective wavelengths near the wavelength of 540 nm, near the wavelength of 600 nm, and near the wavelength of 630 nm may be respectively allocated to the B, G, and R channels. Specifically, an image obtained by allocating the image near the wavelength of 540 nm to the B channel, allocating the image near the wavelength of 600 nm to the G channel, and allocating the image near the wavelength of 630 nm to the R channel may be displayed.

Further, after the structure enhancement processing and predetermined interband calculation are carried out for each of the monochrome images, the images may be allocated to channels corresponding to the images.

The two modifications explained in the first embodiment can also be applied to the endoscope apparatus according to the second embodiment. Specifically, three narrow band lights can also be generated using a light emitting device such as an LED as the light source. Three narrow band lights may be generated by the spectral estimation section.

Therefore, with the endoscope apparatuses according to the embodiments and the modifications of the embodiments explained above, the relatively thick blood vessel running from the submucosa to the muscularis propria is displayed at high contrast. Consequently, the surgeon can accurately grasp a position of such a blood vessel and apply surgery such as the ESD. The blood vessel having the diameter of 1 to 2 mm running from the submucosa to the muscularis propria is displayed on the observation monitor with high visibility. Consequently, the surgeon can dissect a peripheral portion of a lesioned part with an electric knife while paying attention to such a blood vessel. Therefore, in a surgery such as the ESD, it is possible to prevent the surgeon from cutting such a blood vessel by mistake. This leads to a further substantial reduction in a bleeding risk during surgery than in the past.

As explained above, according to the embodiments and the modifications explained above, it is possible to realize an endoscope apparatus and a method of displaying an object image using an endoscope that can clearly display a blood vessel in a deep portion of a mucous membrane without performing complicated work of drug administration.

The present invention is not limited to the embodiments explained above. It goes without saying that various alternations and modifications are possible.

**Claims**

1. An endoscope apparatus (1; 1B) comprising:

an illuminating section (51) that is configured to alternately illuminate an object to be examined with, as illumination light, light in a first wavelength band and light in a second wavelength band, which are in a red band of a visible region and in a wavelength band, where a hemoglobin light absorption characteristic is attenuated by a predetermined amount or more, the light in the first wavelength band being narrow band light having a center wavelength of 600 nm, the light in the second wavelength band being narrow band light having a center wavelength of 630 nm;

an image pickup section (2, 52) that is configured to receive reflected light of the illumination light irradiated to the object to be examined by the illuminating section (51) and to generate a first image pickup signal (P1) corresponding to the first wavelength band and a second image pickup signal (P2) corresponding to the second wavelength band, the apparatus being **characterized in that** it further comprises

a first transformation section (71; 101; 101B) that is an orthogonal transformation section (71) and is configured to generate an average image signal (PA) of the first image pickup signal (P1) and the second image pickup signal (P2), the average image signal (PA) being weighted and not being a simple average of the first image pickup signal (P1) and the second image pickup signal (P2), based on the first image pickup signal (P1) and the second image pickup signal (P2) generated by the image pickup section (52), and is configured to generate a difference image signal (PS) of the first and second image pickup signals (P1, P2), the difference image signal (PS) being weighted and not being a simple difference between the first and second image pickup signals (P1, P2), by orthogonal transformation for each of corresponding pixels of the first and second image pickup signals (P1, P2) through matrix calculation using a coefficient matrix (A);

a first filter section (72; 101; 101B) that is a band-pass filter (72) and is configured to generate from the average image signal (PA) generated by the first transformation section (71; 101; 101B), a first image signal, in which a first spatial frequency component (AR1) corresponding to a blood vessel having a thickness of 1 to 2 mm in the object to be examined is emphasized and a second spatial high-frequency component (AR2), which is higher than the first spatial frequency component and corresponds to a capillary vessel, is suppressed;

a second filter section (73; 101; 101B) that is a low-pass filter (73) and is configured to generate a second image signal, in which the second spatial high-frequency component (AR2), which is higher than the first spatial frequency component (AR1) corresponding to the blood vessel is suppressed with respect to the difference image signal (PS) generated by the first transformation section (71; 101; 101B);

a second transformation section (74; 101; 101B) that is an inverse orthogonal transformation section (74) and is configured to generate a third image signal (P11) corresponding to the first wavelength band and a fourth image signal (P12) corresponding to the second wavelength band from the first image signal generated by the first filter section (72; 101; 101B) and the second image signal generated by the second filter section (73; 101; 101B) by inverse orthogonal transformation for each of corresponding pixels of the first and second image signals generated by the first and second filter sections (72, 73; 101; 101B) through matrix calculation using an inverse matrix (B) of the coefficient matrix (A); and

a display (5) that is configured to allocate the third image signal (P11) to green (G) and blue (B) channels and the fourth image signal (P12) to a red (R) channel, and to display the image signals.

**2.** The endoscope apparatus (1; 1B) according to claim 1,
further comprising a structure enhancement processing section that is configured to execute structure enhancement processing on a fifth image signal generated based on a third image pickup signal obtained by the image pickup section (2, 52), the third image pickup signal corresponding to a third wavelength band in a wavelength band shorter than the first and second wavelength bands,
wherein, when the fifth image signal is generated, a display section allocates the third image signal to the red channel and the fifth image signal to the green and blue channels, and displays the image signals.

**Patentansprüche**

**1.** Endoskopvorrichtung (1; 1B), umfassend:

einen Beleuchtungsteil (51), der dazu eingerichtet ist, ein zu untersuchendes Objekt abwechselnd zu beleuchten mit, als Beleuchtungslicht, Licht in einem ersten Wellenlängenband und Licht in einem zweiten Wellenlängenband, die in einem roten Band eines sichtbaren Bereichs und in einem Wellenlängenband liegen, wo eine Hämoglobin-Lichtabsorptionseigenschaft um eine vorbestimmte Menge oder mehr verringert ist, wobei das Licht in dem ersten Wellenlängenband schmalbandiges Licht mit einer Zentralwellenlänge von 600 nm ist, wobei das Licht in dem zweiten Wellenlängenband schmalbandiges Licht mit einer Zentralwellenlänge von 630 nm ist;
einen Bildaufnahmeteil (2, 52), der dazu eingerichtet ist, reflektiertes Licht des von dem Beleuchtungsteil (51) auf das zu untersuchende Objekt gestrahlten Beleuchtungslichts zu empfangen und ein erstes Bildaufnahmesignal (P1), das dem ersten Wellenlängenband entspricht, und ein zweites Bildaufnahmesignal (P2), das dem zweiten Wellenlängenbereich entspricht, zu erzeugen,

wobei die Vorrichtung **dadurch gekennzeichnet ist, dass** sie ferner umfasst

einen ersten Transformationsteil (71; 101; 101B), der ein orthogonaler Transformationsteil (71) ist und dazu eingerichtet ist, ein Durchschnittsbildsignal (PA) des ersten Bildaufnahmesignals (P1) und des zweiten Bildaufnahmesignals (P2), wobei das Durchschnittsbildsignal (PA) gewichtet ist und nicht einen einfachen Durchschnitt des ersten Bildaufnahmesignals (P1) und des zweiten Bildaufnahmesignals (P2) darstellt, basierend auf dem ersten Bildaufnahmesignal (P1) und dem zweiten Bildaufnahmesignal (P2), die von dem Bildaufnahmeteil (52) erzeugt werden, zu erzeugen, und dazu eingerichtet ist, ein Differenzbildsignal (PS) des ersten und zweiten Bildaufnahmesignals (P1, P2), wobei das Differenzbildsignal (PS) gewichtet ist und nicht eine einfache Differenz zwischen dem ersten und zweiten Bildaufnahmesignal (P1, P2) darstellt, durch orthogonale Transformation für jeden der entsprechenden Pixel des ersten und zweiten Bildaufnahmesignals (P1, P2) durch Matrixberechnung unter Verwendung einer Koeffizientenmatrix (A) zu erzeugen;
einen ersten Filterteil (72; 101; 101B), der ein Bandpassfilter (72) ist und dazu eingerichtet ist, aus dem Durchschnittsbildsignal (PA), das von dem ersten Transformationsteil (71; 101; 101B) erzeugt wird, ein erstes Bildsignal zu erzeugen, in welchem eine erste Ortsfrequenzkomponente (AR1), die einem Blutgefäß mit einer Dicke von 1 bis 2 mm in dem zu untersuchenden Objekt entspricht, hervorgehoben wird und eine zweite Ortshochfrequenzkomponente (AR2), die höher ist als die erste Ortsfrequenzkomponente und einem Kapillargefäß entspricht, unterdrückt wird;
einen zweiten Filterteil (73; 101; 101B), der ein Tiefpassfilter (73) ist und dazu eingerichtet ist, ein zweites Bildsignal zu erzeugen, in welchem die zweite Ortshochfrequenzkomponente (AR2), die höher ist als die erste dem Blutgefäß entsprechende Ortsfrequenzkomponente (AR1), in Bezug auf das von dem ersten Transformationsteil (71; 101; 101B) erzeugten Differenzbildsignal (PS) unterdrückt wird;
einen zweiten Transformationsteil (74; 101; 101B), der ein inverser orthogonaler Transformationsteil (74) ist und dazu eingerichtet ist, ein drittes Bildsignal (P11), das dem ersten Wellenlängenband entspricht, und ein viertes Bildsignal (P12), das dem zweiten Wellenlängenbereich entspricht, aus dem von dem ersten Filterteil (72; 101; 101B) erzeugten ersten Bildsignal und dem von dem zweiten Filterteil (73; 101; 101B) erzeugten zweiten Bildsignal durch inverse orthogonale Transformation für jeden der entsprechenden Pixel des ersten und zweiten Bildsignals, die von dem ersten und zweiten Filterteil (72, 73; 101; 101B) erzeugt werden, durch Matrixberechnung unter Verwendung einer inversen Matrix (B) der Koeffizientenmatrix (A) zu erzeugen; und
ein Display (5), das dazu eingerichtet ist, das dritte Bildsignal (P11) grünen (G) und blauen (B) Kanälen und das vierte Bildsignal (P12) einem roten (R) Kanal zuzuteilen, und die Bildsignale anzuzeigen.

**2.** Endoskopvorrichtung (1; 1B) nach Anspruch 1,
ferner umfassend einen Strukturverstärkungsverarbeitungsteil, der dazu eingerichtet ist, Strukturverstärkungsverarbeitung auf einem fünften Bildsignal auszuführen, das basierend auf einem dritten Bildaufnahmesignal, welches von dem Bildaufnahmeteil (2, 52) erhalten wird, erzeugt wird, wobei das dritte Bildaufnahmesignal einem dritten Wellenlängenband in einem Wellenlängenband entspricht, das kürzer als das erste und zweite Wellenlängenband ist, wobei, wenn das fünfte Bildsignal erzeugt ist, ein Anzeigeteil das dritte Bildsignal dem roten Kanal und das fünfte Bildsignal den grünen und blauen Kanälen zuteilt, und die Bildsignale anzeigt.

## Revendications

1. Appareil d'endoscope (1 ; 1B) comprenant :

   une section d'éclairage (51) qui est configurée pour éclairer alternativement un objet devant être examiné avec, en tant que lumière d'éclairage, une lumière dans une première bande de longueur d'onde et une lumière dans une deuxième bande de longueur d'onde, qui sont dans une bande rouge d'une région visible et dans une bande de longueur d'onde, dans laquelle une caractéristique d'absorption de la lumière par l'hémoglobine est atténuée d'une quantité prédéterminée ou plus, la lumière dans la première bande de longueur d'onde étant une lumière à bande étroite présentant une longueur d'onde centrale de 600 nm, la lumière dans la deuxième bande de longueur d'onde étant une lumière à bande étroite présentant une longueur d'onde centrale de 630 nm ;
   une section de capture d'image (2, 52) qui est configurée pour recevoir la lumière réfléchie de la lumière d'éclairage rayonnée sur l'objet devant être examiné par la section d'éclairage (51) et générer un premier signal de capture d'image (P1) correspondant à la première bande de longueur d'onde et un deuxième signal de capture d'image (P2) correspondant à la deuxième bande de longueur d'onde,
   l'appareil étant **caractérisé en ce qu'**il comprend en outre
   une première section de transformation (71 ; 101 ; 101B) qui est une section de transformation orthogonale (71) et est configurée pour générer un signal d'image moyen (PA) du premier signal de capture d'image (P1) et du deuxième signal de capture d'image (P2), le signal d'image moyen (PA) étant pondéré et n'étant pas une simple moyenne du premier signal de capture d'image (P1) et du deuxième signal de capture d'image (P2), sur la base du premier signal de capture d'image (P1) et du deuxième signal de capture d'image (P2) générés par la section de capture d'image (52), et est configurée pour générer un signal d'image de différence (PS) des premier et deuxième signaux de capture d'image (P1, P2), le signal d'image de différence (PS) étant pondéré et n'étant pas une simple différence entre les premier et deuxième signaux de capture d'image (P1, P2), par transformation orthogonale pour chacun des pixels correspondants des premier et deuxième signaux de capture d'image (P1, P2) par l'intermédiaire d'un calcul matriciel utilisant une matrice de coefficient (A) ;
   une première section de filtre (72 ; 101 ; 101B) qui est un filtre passe-bande (72) et est configurée pour générer à partir du signal d'image moyen (PA) généré par la première section de transformation (71 ; 101 ; 101B), un premier signal d'image, dans lequel une première composante de fréquence spatiale (AR1) correspondant à un vaisseau sanguin présentant une épaisseur de 1 à 2 mm dans l'objet devant être examiné est accentuée et une deuxième composante de fréquence spatiale élevée (AR2), qui est plus élevée que la première composante de fréquence spatiale et correspond à un vaisseau capillaire, est supprimée ;
   une deuxième section de filtre (73 ; 101 ; 101B) qui est un filtre passe-bas (73) et est configurée pour générer un deuxième signal d'image, dans lequel la deuxième composante de fréquence spatiale élevée (AR2), qui est plus élevée que la première composante de fréquence spatiale (AR1) correspondant au vaisseau sanguin est supprimée par rapport au signal d'image de différence (PS) généré par la première section de transformation (71 ; 101 ; 101B) ;
   une deuxième section de transformation (74 ; 101 ; 101B) qui est une section de transformation orthogonale inverse (74) et est configurée pour générer un troisième signal d'image (P11) correspondant à la première bande de longueur d'onde et un quatrième signal d'image (P12) correspondant à la deuxième bande de longueur d'onde à partir du premier signal d'image généré par la première section de filtre (72 ; 101 ; 101B) et du deuxième signal d'image généré par la deuxième section de filtre (73 ; 101 ; 101B) par transformation orthogonale inverse pour chacun des pixels correspondants des premier et deuxième signaux d'image générés par les première et deuxième sections de filtre (72, 73 ; 101 ; 101B) par l'intermédiaire d'un calcul matriciel utilisant une matrice inverse (B) de la matrice de coefficient (A) ; et
   un affichage (5) qui est configuré pour attribuer le troisième signal d'image (P11) aux canaux vert (G) et bleu (B) et le quatrième signal d'image (P12) à un canal rouge (R), et afficher les signaux d'image.

2. Appareil d'endoscope (1 ; 1B) selon la revendication 1,
   comprenant en outre une section de traitement d'amélioration de structure qui est configurée pour exécuter un traitement d'amélioration de structure sur un cinquième signal d'image généré sur la base d'un troisième signal de capture d'image obtenu par la section de capture d'image (2, 52), le troisième signal de capture d'image correspondant à une troisième bande de longueur d'onde dans une bande de longueur d'onde plus courte que les première et deuxième bandes de longueur d'onde,

dans lequel, lorsque le cinquième signal d'image est généré, une section d'affichage attribue le troisième signal d'image au canal rouge et le cinquième signal d'image aux canaux vert et bleu, et affiche les signaux d'images.

**FIG.1**

# FIG.2

# FIG.3

# FIG.4

FIRST IMAGE (λ1 = 600 nm) — P1

SECOND IMAGE (λ2 = 630 nm) — P2

101

ORTHOGONAL TRANSFORMATION SECTION — 71

BPF — 72

LPF — 73

INVERSE ORTHOGONAL TRANSFORMATION SECTION — 74

TO R CHANNEL P12 — SECOND IMAGE (λ2 = 630 nm)

TO G CHANNEL P11 — FIRST IMAGE (λ1 = 600 nm)

TO B CHANNEL P11 — FIRST IMAGE (λ1 = 600 nm)

SS

# FIG.5

# FIG.6

# FIG.7

EP 2 604 170 B1

# FIG.8

# FIG.9

# FIG.10

FIRST IMAGE
(λ1 = 600 nm)    P1

SECOND IMAGE
(λ2 = 630 nm)    P2

THIRD IMAGE
(λ3 = 540 nm)    P3

ORTHOGONAL
TRANSFORMATION
SECTION    71

BPF    72

LPF    73

INVERSE
ORTHOGONAL
TRANSFORMATION
SECTION    74

SE    75

TO R CHANNEL    P11
FIRST IMAGE
(λ1 = 600 nm)

TO G CHANNEL    P13
THIRD IMAGE
(λ3 = 540 nm)

TO B CHANNEL    P13
THIRD IMAGE
(λ3 = 540 nm)

101B

SS

EP 2 604 170 B1

# FIG.11

**EP 2 604 170 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1787577 A1
- JP 2000041942 A